# EUROPÄISCHE PATENTSCHRIFT

(11) **EP 1 617 819 B1**
(45) Veröffentlichungstag und Bekanntmachung des Hinweises auf die Patenterteilung: **17.08.2011**
(21) Anmeldenummer: 04725904.9
(22) Anmeldetag: 06.04.2004
(51) Int. Cl.: A61K 9/16, A61K 49/00, A61K 51/12, A61K 9/00

(54) **RADIOAKTIV MARKIERTE MIKROPARTIKEL, VERFAHREN ZU DEREN HERSTELLUNG UND DEREN VERWENDUNG**
RADIOACTIVELY MARKED MICROPARTICLES, METHOD FOR THE PRODUCTION THEREOF AND USE OF THE SAME
MICROPARTICULES A MARQUAGE RADIOACTIF, PROCEDE DE PRODUCTION ET UTILISATION

(30) Priorität: 16.04.2003 DE 10317461
(43) Veröffentlichungstag der Anmeldung: 25.01.2006
(60) Teilanmeldung: 10180824.4
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: HARTIG-HEIMEL Mareke, 55218 Ingelheim (DE); TRUNK, Michael, 55218 Ingelheim (DE); WEUTHEN, Thomas, 89420 Höchstädt (DE)
(86) Internationale Anmeldenummer: PCT/EP2004/003644
(87) Internationale Veröffentlichungsnummer: WO 2004/091581

(56) Entgegenhaltungen:
- DE-A- 2 127 809
- DE-A- 19 732 323
- GB-A- 1 269 383
- US-A- 3 663 687
- US-A- 3 810 976
- US-A- 3 863 004
- DELGADO A ET AL: "Radiolabelled biodegradable microspheres for lung imaging" EUROPEAN JOURNAL OF PHARMACEUTICS AND BIOPHARMACEUTICS, ELSEVIER SCIENCE PUBLISHERS B.V., AMSTERDAM, NL, Bd. 50, Nr. 2, September 2000 (2000-09), Seiten 227-236, XP004257195 ISSN: 0939-6411
- MOLLER W ET AL: "Preparation of spherical monodisperse ferrimagnetic iron-oxide microparticles between 1 and 5mum diameter" JOURNAL OF MAGNETISM AND MAGNETIC MATERIALS, ELSEVIER SCIENCE PUBLISHERS, AMSTERDAM, NL, Bd. 225, Nr. 1-2, 2001, Seiten 8-16, XP004234916 ISSN: 0304-8853

## Beschreibung

Die Erfindung betrifft mit dem Technetium-Isotop ^{99m}Tc radioaktiv markierte Mikropartikel, ein Verfahren zu deren Herstellung, sowie deren Verwendung zur Herstellung von - für beispielsweise für Depositionsstudien geeigneten - Pulverinhalativa, die die markierten Mikropartikel als Wirkstoffe enthalten.

Die nach diesem Verfahren hergestellten radioaktiven Pulverinhalativa unterscheiden sich weder hinsichtlich ihrer physiko-chemischen und technischen Eigenschaften noch in ihrer medizinischen Anwendung von den entsprechenden nicht markierten / analogen Pulverinhalativa, die großtechnisch als Arzneimittel hergestellt werden (Marktware).

### Hintergrund der Erfindung

Bei der Applikationsform "Pulverinhalativum" werden Inhalationspulver, die beispielsweise in geeignete Kapseln (Inhaletten) abgefüllt werden, mittels Pulverinhalatoren ausgebracht. Alternativ dazu kann eine inhalative Anwendung auch durch Applikation geeigneter pulverförmiger Inhalationsaerosole, die beispielsweise HFA134a, HFA227 oder Gemische aus diesen als Treibgas enthalten, erfolgen.
Üblicherweise werden Pulverinhalativa, z.B. in Form von Kapseln zur Inhalation, auf Basis der allgemeinen Lehre, wie sie in DE-A-179 22 07 beschrieben ist, hergestellt. Ein bedeutender Aspekt bei der inhalativen Applikation eines Wirkstoffes ist, dass nur Teilchen einer bestimmten aerodynamischen Größe in das Zielorgan, die Lunge, gelangen. Die Teilchengröße dieser lungengängigen Partikel (inhalierbarer Anteil) liegt im Bereich weniger Mikrometer. Solche Partikel werden üblicherweise durch Mikronisierung (z.B. Luftstrahlmahlung) erzeugt. Neben dem Strahlmahlverfahren ist es auch möglich, ein geeignetes Mikronisat über Alternatiwerfahren herzustellen. Literatur-bekannt ist dabei die Herstellung von inhalierbaren Mikropartikeln mittels Sprühtrocknung. Üblicherweise werden aus solchen Sprühtrocknungspartikeln wiederum in Anlehnung an oben zitiertes Verfahren (DE-A-179 22 07) technisch handhabbare Formulierungen hergestellt, die eine ausreichende Dispergierbarkeit bei der medizinischen Anwendung (Inhalation) aufweisen [Y.-F. Maa, P,-A. Ngyuyen, J.D. Andya, N. Dasovich, T.D. Sweeny, S.J. Shire, C.C. Hsu, Pharmaceutical Research, 15, No. 5 (1998), 768-775; M.T. Vidgrén, P.A. Vidgrén, T.P. Paronen, Int. J. Pharmaceutics, 35 (1987), 139-144; R.W. Niven, F.D. Lott, A.Y. Ip, J.M. Cribbs, Pharmaceutical Research, 11, No. 8 (1994), 1101-1109]. Bei derartigen Formulierungen ist u.a. eine gleichmäßige Verteilung des Arzneimittels in der Pulvermischung (Formulierung) ein kritischer Faktor.

Zum Nachweis der Wirksamkeit und des Wirkortes eines Arzneimittels - insbesondere bei der inhalativen Applikation - kann eine Depositionstudie beitragen, bei der nach radioaktiver Markierung des Wirkstoffes dessen Verteilung im menschlichen Körper szintigraphisch detektiert wird. Um eine eindeutige, quantitative Korrelation zwischen Wirkstoffverteilung und radioaktiver Verteilung zu garantieren, müssen der Markierungsprozeß und die Formulierung so gestaltet sein, dass die quantitative Lungendeposition des Wirkstoffes genau der quantitativen Verteilung der Radioaktivität entspricht.

Für Lösungsaerosole, die in Form feinster Mikrotröpfchen inhaliert werden, ergibt sich - eine homogene Wirkstoffverteilung vorausgesetzt - als technische Voraussetzung, dass die radioaktive Substanz in gleicher Weise homogen in der zu versprühenden Lösung verteilt sein muß. Üblicherweise wird dies durch homogenes Lösen der radioaktiven Substanz in der Wirkstofflösung erreicht [K.P. Steed, L.J. Towse, B. Freund, S.P. Newman; Eur. J. Pharm. Sci. (1997), 5 (2), 55-61].

Die prinzipielle Vorgehensweise bei der radioaktiven Markierung von Treibgasgetriebenen MDIs unterscheidet sich nur gering von dem Markierungsverfahren für Vernebler-Formulierungen: In der Suspensionsformulierung, bestehend aus einem mikronisierten Wirkstoff, der als Feststoffpartikel vorliegt, und dem unter Druck stehenden Treibgas, das in flüssiger Phase vorliegt, wird die radioaktive Substanz im flüssigen Treibgasmedium homogen gelöst. Während der Anwendung (Zerstäubung) verdunstet das Treibgas in den durch den Sprühvorgang entstehenden Mikrotröpfchen, die aus Treibgas (flüssig), radioaktiver Substanz (gelöst) und mikronisiertem Wirkstoff (suspendiert) bestehen, spontan, und die radioaktive Substanz schlägt sich an den Wirkstoff-Mikropartikeln nieder. Auch bei dieser Vorgehensweise wird eine direkte quantitative Korrelation zwischen Wirkstoff und radioaktiver Markierung gewährleistet [S.P. Newman, A.R. Clark, N. Talaee, S.W. Clarke; Thorax (1989), 44, 706-710; R. Pauwels, S. Newman, L. Bergstrom; Eur.Respiratory J. (1997), 10 (9), 2127-2138].

Die oben beschriebenen Vorgehensweisen zur radioaktiven Wirkstoffmarkierung sind nicht auf Pulverformulierungen übertragbar, sofern diese aus einer Pulvermischung mit mindestens einem pharmakologisch nicht-wirksamen Trägermaterial (Feststoff) und mindestens einem mikronisierten Wirkstoff (Feststoff) bestehen. Für solche Systeme muß neben der oben genannten quantitativen Korrelation zwischen Radioaktivität und Wirkstoff, bezogen auf die Partikelgröße und -masse, zusätzlich gewährleistet sein, dass der Radiomarker selektiv / spezifisch den Wirkstoff markiert und nicht auch das Trägermaterial.

Es ist Aufgabe der vorliegenden Erfindung, radioaktiv markierte Mikropartikel bereitzustellen, die im Rahmen von inhalativ applizierbaren Pulverformulierungen zum Einsatz gelangen können und der vorstehend genannten Anforderung gerecht werden.

### Detaillierte Beschreibung der Erfindung

Die Erfindung betrifft mit dem Technetiumisotop ^{99m}Tc radioaktiv markierte Mikropartikel, das Verfahren zur Herstellung solcher Mikropartikel sowie die Verwendung solcher Mikropartikel zur Herstellung einer stabilen Pulver-Formulierung. Die erfindungsgemäßen Mikropartikel werden den vorstehend genannten Anforderungen gerecht.

Nachfolgend wird statt der Bezeichnung "Technetiumisotop ^{99m}Tc" auch die Abkürzung Tc* verwendet.

Die erfindungsgemäßen Formulierungen unterscheiden sich bezüglich der pharmazeutischen Eigenschaften wie z. B. des aerodynamischen Verhaltens und der Dispergierbarkeit nicht von den pharmazeutisch zum Einsatz gelangenden Formulierungen, in denen der Wirkstoff nicht markiert ist.

Die nach dem erfindungsgemäßen Verfahren hergestellten radioaktiv-markierten Mikropartikel sowie die daraus hergestellten Pulver-Formulierungen für inhalative Zwecke erweisen sich als ausreichend stabil, um damit sogenannte Depositionsstudien durchführen zu können.

Unter Mikropartikeln werden im Rahmen der vorliegenden Erfindung pharmazeutische Wirkstoffe in fester, vorzugsweise kristalliner Form verstanden, die eine mittlere Teilchengröße von etwa 0,5 bis etwa 10 µm, bevorzugt von etwa 1 bis etwa 6 µm, besonders bevorzugt von etwa 1,5 bis etwa 5 µm aufweisen. Dabei wird unter der mittleren Teilchengröße im hier verwendeten Sinne der 50 % - Wert aus der Volumenverteilung, gemessen mittels Laserbeugung nach der Trockendispersionsmethode, verstanden. Methoden zur Bestimmung der mittleren Teilchengröße sind aus dem Stand der Technik bekannt. Beispielsweise sei auf die diesbezüglichen Ausführungen im experimentellen Teil der WO 02/30389 hingewiesen. Partikel der vorstehend genannten mittleren Teilchengröße werden beispielsweise durch Mahlen (sogenanntes Mikronisieren) von Kristallen größerer Teilchengröße, durch spezielle Kristallisationsverfahren oder auch durch Sprühtrocknungsverfahren generiert. All diese Verfahren sind im Stand der Technik bekannt.

Die vorliegende Erfindung betrifft Verfahren zur Herstellung radioaktiv markierter Mikropartikel pharmazeutischer Wirkstoffe bevorzugt aus der Gruppe der Anticholinergika, Betamimetica, Dopaminagonisten, Antiallergika, Leukotrien-Antagonisten und Corticosteroiden, sowie gegebenenfalls Wirkstoffkombinationen davon.

Die vorliegende Erfindung betrifft ferner radioaktiv markierte Mikropartikel pharmazeutische Wirkstoffe aus der Gruppe der Anticholinergika erhältlich nach erfindungsgemäßen Verfahren im Gemisch mit einem oder mehreren geeigneten, physiologisch unbedenklichen Hilfsstoffen sowie deren Verwendung.

Als Anticholinergika kommen Verbindungen ausgewählt aus der Gruppe der Tiotropiumsalze, Ipratropiumsalze, Oxitropiumsalze,
Salze der aus der WO 02/32899 bekannten Verbindungen
N-Methyl-2,2-Diphenylpropionsäuretropenolester,
N-Methyl-2,2-Diphenylpropionsäurescopinester,
N-Methyl-2-Fluor-2,2-Diphenylessigsäurescopinester und
N-Methyl-2-Fluor-2,2-Diphenylessigsäuretropenolester
sowie Salze der aus der WO 02/32899 bekannten Verbindungen
N-Methyl-3,3',4,4'-Tetrafluorbenzilsäuretropenolester,
N-Methyl-3,3',4,4'-Tetrafluorbenzilsäurescopinester;
N-Methyl-4,4'-Dichlorbenzilsäurescopinester,
N-Methyl-4,4'-Difluorbenzilsäurescopinester,
N-Methyl-3,3'-Difluorbenzilsäuretopenolester,
N-Methyl-3,3'-Difluorbenzilsäurescopinester und
N-Ethyl-4,4'-Difluorbenzilsäuretropenolester gegebenenfalls in Form ihrer Hydrate und Solvate, in Betracht.
Unter Salzen sind dabei diejenigen Verbindungen zu verstehen, die neben den vorstehend genannten Kationen als Gegenion ein einfach negativ geladenes Anion ausgewählt aus der Gruppe Chlorid, Bromid und Methansulfonat enthalten.

Besonders bevorzugt kommen als Wirkstoffe im Rahmen der vorliegenden Erfindung die Bromide oder Methansulfonate der vorstehend genannten Strukturen in Betracht. Von herausragendem Interesse sind im Rahmen der vorliegenden Erfindung beispielsweise Anticholinergika Tiotropiumbromid, Ipratropiumbromid, Oxitropiumbromid, 2,2-Diphenylpropionsäuretropenolester-methobromid, 2,2-Diphenylpropionsäurescopinester-methobromid, 2-Fluor-2,2-Diphenylessigsäure-scopinester-methobromid, 2-Fluor-2,2-Diphenylessigsäuretropenolester-methobromid, 3,3',4,4'-Tetrafluorbenzilsäuretropenolester-methobromid, 3,3',4,4'-Tetrafluorbenzilsäurescopinester-methobromid; 4,4'-Dichlorbenzilsäurescopinestermethobromid, 4,4'-Difiuorbenzilsäurescopinester-methobromid, 3,3'-Difluorbenzilsäuretropenolester-methobromid, 3,3'-Difluorbenzilsäurescopinester-methobromid und 4,4'-Difluorbenzilsäuretropenolester-ethylbromid woebi der Tiotropiumbromid, dem Ipratropiumbromid, dem 2,2-Diphenylpropionsäuretropenolester-methobromid, dem 2,2-Diphenylpropionsäurescopinester-methobromid, dem 2-Fluor-2,2-Diphenylessigsäurescopinester-methobromid sowie dem 2-Fluor-2,2-Diphenylessigsäuretropenolester-methobromid besondere Bedeutung zukommen. Von herausragender Bedeutung ist hierbei das Tiotropiumbromid, besonders bevorzugt in Form seines aus der WO 02/30928 bekannten kristallinen Monohydrats.

Als Betamimetika kommen erfindungsgemäß bevorzugt diejenigen Verbindungen in Betracht, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salbutamol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol und 1-(4-Ethoxycarbonylamino-3-cyano-5-fluorophenyl)-2-(tert.-butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Besonders bevorzugt gelangen als Betamimetika solche Wirkstoffe zur Anwendung, die ausgewählt sind aus der Gruppe bestehend aus Fenoterol, Formoterol, Salmeterol, Salbutamol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.
Beispeilsweise sind die Säureadditionssalze der Betamimetika ausgewählt aus der Gruppe bestehend aus Hydrochlorid, Hydrobromid, Sulfat, Phosphat, Fumarat Methansulfonat und Xinafoat.
Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.
Besonders bevorzugt sind die Salze im Falle des Salmeterols ausgewählt aus Hydrochlorid, Sulfat und Xinafoat, von denen das Xinafoat besonders bevorzugt ist.

Im Rahmen der vorliegenden Erfindung werden unter Corticosteroiden, die erfindungsgemäß zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Desloratidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Das erfindungsgemäße Verfahren zur Herstellung von mit dem Technetium-Isotop ^{99m}Tc Tc*) radioaktiv markierten Mikropartikel ist dadurch gekennzeichnet, dass ein Tc*-Salz in einem Lösungsmittel aufgenommen wird, diese Lösung mit in einem Suspensionsmittel suspendierten Mikropartikeln versetzt und gemischt wird und abschließend das Suspensionsmittel und das Lösungsmittel entfernt werden.

Die erfindungsgemäßen, mit dem Technetium-Isotop ^{99m}Tc (Tc*) radioaktiv markierten Mikropartikel können durch ein Verfahren hergestellt werden, welches erfindungsgemäß dadurch gekennzeichnet ist, dass ein Tc*-Salz in einem Lösungsmittel aufgenommen wird, diese Lösung mit in einem Suspensionsmittel suspendierten Mikropartikeln versetzt und gemischt wird und abschließend das Suspensionsmittel und das Lösungsmittel entfernt werden.

Im Rahmen der vorliegenden Erfindung beziehen sich die Bezeichnungen Lösungs- und Suspensionsmittel auf die Löseeigenschaften hinsichtlich des zu markierenden Wirkstoffs.

Die Darstellung der erfindungsgemäßen, radioaktiv markierten Mikropartikel kann dabei beispielsweise wie folgt erfolgen.
Zur radioaktiven Markierung der Mikropartikel wird nach bekannten Verfahren zuerst das radioaktive Tc* aus einer wässrigen Lösung gewonnen. Beispielweise kann dabei das Technetium in Form von Pertechnetat-Ionen (TcO₄⁻) aus einem Tc-Generator mit NaCl-Lösung eluiert und nach Eindampfen der wässrigen Lösung bis zur Trockene bereitgestellt werden. Dabei liegt das Pertechnetat-Salz NaTc*O₄ im Gemisch mit NaCl vor.

Das hier beschriebene System zur Markierung eines Pulvermikronisats basiert auf der Vorgehensweise, dass radioaktives Tc* in Lösung gebracht, gegebenenfalls mit einem (organischen) Suspensionsmittel (bezogen auf den zu markierenden Wirkstoff nach obiger Definition) gemischt und in dieser Lösung bzw. diesem Lösungs-/Suspensionsmittelgemisch der mikronisierte Wirkstoff suspensiert wird. Zur Gewinnung der markierten Partikel wird dann das Lösungsmittel bzw. das Lösungs-/Suspensionsmittelgemisch z.B. durch Verdampfung entfernt, so dass sich das Tc*-Salz auf der Oberfläche der Mikropartikel niederschlägt.

Unter radioaktiv markierten Mikropartikeln im Sinne der vorliegenden Erfindung sind dementsprechend solche Mikropartikel zu verstehen, auf deren Oberfläche radioaktives Tc*, insbesondere in Form eines Tc*-Salzes, niedergeschlagen ist.

Unter Tc*-Salz im Sinne der vorliegenden Erfindung sind Salze des Technetiumisotop ^{99m}Tc zu verstehen, die vorzugsweise ausgewählt sind aus der Gruppe bestehend aus NaTc*O₄, ^{99m}Tc-TPAC (Tetraphenylarsomiumpertechnetat) und ^{99m}Tc-DTPA (Diethylenetriaminepentaacetacid), gegebenenfalls im Gemisch mit NaCl.

Überraschenderweise wurde gefunden, dass sich eine gleichförmige Suspension des Mikronisats im Suspensionsmittel durch die Zugabe einer geringen Menge eines Lösungsmittels, in dem sich der zu suspendierende Wirkstoff prinzipiell lösen lässt, herstellen lässt und dass nach Verdampfung des Lösungs-/Suspensionsmittelgemischs Mikropartikel entstehen, die sich in ihren kristallinen Eigenschaften nicht von dem eingesetzten Ausgangsmikronisat unterscheiden. In bevorzugter Art und Weise wird das vorstehend genannte Lösungsmittel so gewählt, dass es ebenfalls zur Lösung des zu verwendenden Tc*-Salzes befähigt ist.

Das zur Trockene eingedampfte Tc*-Salz, besonders bevorzugt in Form des Salzgemisches NaTc*O₄ / NaCl, wird in einem Lösungsmittel aufgenommen, das sich durch gute Löseeigenschaften für das Tc*-Salz auszeichnet. Als besonders geeignet haben sich dabei Alkohole, Ether, Ketone, Halogenkohlenwasserstoffe, polare organische Lösungsmittel oder Gemische der vorstehend genannten Lösungsmittel erwiesen. Im Falle der Verwendung eines Alkohols ist dieser bevorzugt ausgewählt aus der Gruppe bestehend aus Methanol, Ethanol, Propanol, Butanol, Glykol und Isopropanol, besonders bevorzugt ist Ethanol. Im Falle der Verwendung eines Ethers ist dieser bevorzugt ausgewählt aus der Gruppe bestehend aus Diethylether, Methyltert-butyl-ether, Tetrahydrofuran, Dioxan und Diisopropylether. Im Falle der Verwendung eines Ketons ist dieses bevorzugt ausgewählt aus der Gruppe bestehend aus Aceton, Methylethylketon und Diethylketon. Im Falle der Verwendung eines Halogenkohlenwasserstoffs ist dieser bevorzugt ausgewählt aus der Gruppe bestehend aus Dichlormethan oder Chloroform, bevorzugt ist Dichlormethan. Im Falle der Verwendung eines polaren organischen Lösungsmittels ist dieses bevorzugt ausgewählt aus der Gruppe bestehend aus Dimethylformamid, Dimethylsulfoxid und Acetonitril. Besonders bevorzugt gelangt als Lösungsmittel ein Alkohol, insbesondere Ethanol, zur Anwendung.

Der Lösung des Tc*-Salzes im Lösungsmittel wird im folgenden Verfahrensschritt ein Suspensionsmittel zugegeben, das in bevorzugter Art und Weise so ausgewählt werden muss, dass in Abhängigkeit von der absoluten Menge an Salzen, die in der Lösung vorliegt, eine vollständige Lösung des vorhandenen Tc*-Salzes erzielt wird.

Erfindungsgemäß geeignete Suspensionsmittel sind ausgewählt aus der Gruppe der unpolaren, aprotonische Lösungsmittel bestehend aus der Gruppe der Paraffine und Olefine bevorzugt Cyclohexan, n-Heptan, Pentan und n-Hexan, bevorzugt n-Hexan.

Besonders bevorzugt wird eine solche Menge an Suspensionsmittel zugesetzt, dass das Verhältnis von Lösungsmittel zu Suspensionmittel in einem Bereich zwischen 1:50 und 1:1000, bevorzugt in einem Bereich zwischen etwa 1:100 und 1:500 liegt. Vorstehende Werte beziehen sich auf die jeweiligen Volumenanteile der beiden Komponenten.

Dabei kann zuerst die flüssige Phase mit dem in einem der vorstehend genannten Lösungsmittel gelösten Tc*-Salz hergestellt werden, in die dann die radioaktiv zu markierenden Mikropartikel gegeben werden.
Alternativ dazu kann eine flüssige Phase, in dem das Tc*-Salz gelöst vorliegt und die darüber hinaus das Suspensionsmittel in niedriger Konzentration enthält, hergestellt werden, indem nach Aufnahme des Tc*-Salzes im Lösungsmittel eine Teilmenge des Suspensionsmittels dieser Lösung zugegeben wird. Anschließend wird diese Lösung mit dem zuvor im restlichen Suspensionsmittel suspendierten Mikronisat vermischt. Nach bekannten Verfahren wird dann das Lösungsmittel-Suspensiansmittel-Gemisch entfernt (z.B. über Verdampfung unter vermindertem Druck). Es verbleiben die erfindungsgemäßen radioaktiv markierten Mikropartikel.

Aus diesen Mikropartikeln lassen sich dann nach aus dem Stand der Technik bekannten Verfahren die gewünschten inhalativ applizierbaren Pulverformulierungen erhalten.
Diese Pulverformulierungen enthalten die erfindungsgemäß radioaktiv markierten Mikropartikel im Gemisch mit einem oder mehereren geeigneten, physiologisch unbedenklichen Hilfsstoffen.

Die folgenden, physiologisch unbedenklichen Hilfsstoffe können dabei erfindungsgemäß zur Anwendung gelangen: Monosaccharide (z.B. Glucose oder Arabinose), Disaccharide (z.B. Lactose, Saccharose, Maltose), Oligo- und Polysaccharide (z.B. Dextrane, Stärke, Stärkederivate, Cellulose, Cellulosederivate), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich, in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 500 µm, bevorzugt zwischen 10 und 250 µm, besonders bevorzugt zwischen 15 und 100 µm auf. Gegebenenfalls kann es sinnvoll sein, den vorstehend genannten Hilfststoffen feinere Hilfsstofffraktionen mit einer mittleren Teilchengröße von etwa 1 bis 9 µm beizumischen. Letztgenannte feinere Hilfsstoffe sind ebenfalls ausgewählt aus der vorstehend genannten Gruppe an einsetzbaren Hilfsstoffen.

Zur Herstellung der Pulverformulierungen werden die erfindungsgemäßen radioaktiv markierten Mikropartikel dem Hilfsstoff, gegebenenfalls der Hilfsstoffmischung, beigemischt. Verfahren zur Herstellung von Inhaltionspulvern durch Mahlen und Mikronisieren sowie durch abschließendes Mischen der Bestandteile sind aus dem Stand der Technik bekannt. Zur Darstellung der erfindungsgemäßen radioaktiv markierten Inhalationspulver bzw. Pulvermischungen, bei denen als pharmazeutischer Wirkstoff ein Tiotropiumsalz zur Anwendung gelangt, wird insbesondere auf die WO 02/30389 verwiesen, insbesondere auf die Ausführungen von Seite 5, Zeile 29, bis Seite 6, Zeile 39, sowie auf die aufgeführten Ausführungsformen der Beispiele 1 bis 4.

Die erfindungsgemäßen Inhalationspulver können mittels aus dem Stand der Technik bekannten Pulverinhalatoren appliziert werden. Diesbezüglich sei beispielsweise auf die Inhalatoren gemäß US 4570630, US 4524769, US4627432, US 5590645, DE 36 25 685 oder auch WO 94/28958 verwiesen.

Die folgenden, detaillierten experimentellen Ausführungen dienen einer weitergehenden Erläuterung der vorliegenden Erfindung, ohne den Umfang der Erfindung auf die nachfolgenden beispielhaften Ausführungsformen zu beschränken.

### Beispiel für die Vorgehensweise der nach obigem Verfahren herstellbaren Mikropartikel (mit Tiotropiumbromid als Wirkstoff)

Folgende Verfahrensschritte beschreiben die Vorgehensweise zur Herstellung von radioaktiv-markierten Mikropartikeln:
1. Mehrere ml einer wäßrigen möglichst hoch konzentrierten Natrium-Pertechnetat-Lösung (= V₁) mit einer Gesamtradioaktivität von bevorzugt 4.500 - 5.000 MBq werden auf einer Heizplatte zur Trockene eingedampft.
2. Der Rückstand wird in Ethanol (= V₂) aufgenommen und auf einer Heizplatte eingeengt bis zum Volumen von ≥ V3.
3. Der Überstand der Lösung über den nicht-gelösten Teilen wird quantitativ abgetrennt (= V₃).
4. In einem Kolben wird mikronisiertes, kristallines Tiotropium Bromid monohydrat (= m₁) und das Suspensionsmittel n-Hexan (= V₄) vorgelegt.
5. Dazu wird die ethanolische Natrium-Pertechnetat-Lösung (= V₃) zugegeben.
6. Von der erhaltenen Mischung werden alle flüssigen Bestandteile beispielsweise bei 43 °C und unter vermindertem Druck von etwa 200 mbar bis zur Trockene abdestilliert.

Der im Kolben verbleibende Rückstand entspricht dem erfindungsgemäß radioaktiv markierten Mikropartikeln und kann zur Herstellung eines radioaktiv markierten Pulverinhalativums weiter verarbeitet werden. Verfahren dazu sind aus dem Stand der Technik bekannt (z.B. WO 02/30390).

In bevorzugter Art und Weise kann im Verfahrensschritt 2 die Lösung so stark eingeengt werden, dass im Verfahrensschritt 3 nur eine kleinere Menge aufgenommen werden kann (z.B. 250µl) und dann durch Wiederholung des Schrittes 2 und 3 das Gesamtvolumen V₃ der Tc*-Ethanol-Lösung entsprechend obiger Angabe hergestellt wird. In einer weiteren Verfahrensvariante kann durch Zugabe (z.B. 10 ml - Teilmenge der Lösung V₄) an reinem Suspensionsmittel zu der aufgenommen Lösung V₃ im Verfahrensschritt 3 das Gesamtvolumen der Tc*-Lösung erhöht werden.

Ebenso erweist sich folgende Vorgehensweise als besonders vorteilhaft: die Suspension nach Verfahrensschritt 4 wird nur unter Verwendung von einer Teilmenge des Suspensionsmittels V₄ hergestellt (z.B. 45 ml). Diesem wird die Häfte der Tc*-Lösung nach obigen bevorzugten Verfahren zugegeben. Nach Einengung dieser Suspension wird die restliche n-Hexan-Menge (z.B. 45 ml) der Suspension zugegeben sowie die restliche Tc*-Lösung dieser Suspension zugemischt. Diese Suspension wird dann entsprechend obigen Verfahrensschritt 6 weiterverarbeitet.

**Tabelle 1: Mengen- und Volumenangaben**

| | Beispiel 1 | Beispiel 2 |
|---|---|---|
| V₁ | 6 ml | 1.5-2 ml |
| V₂ | 10 ml | 2 x 1 ml |
| V₃ | 0.5 ml | 2 x 250 µl |
| V₄ | 99.5 ml | 100 ml |
| m₁ | 2 g | 80 mg |

## Patentansprüche

1. Verfahren zur Herstellung von mit dem Technetium-Isotop ^{99m}Tc (Tc*) radioaktiv markierten Mikropartikeln mit einer mittleren Teilchengröße von etwa 0,5 bis etwa 10 µm, welches **dadurch gekennzeichnet ist, dass**
ein Tc*-Salz in einem Lösungsmittel aufgenommen wird, diese Lösung mit in einem Suspensionsmittel suspendierten Mikropartikeln versetzt und gemischt wird und abschließend das Suspensionsmittel und das Lösungsmittel durch Verdampfung entfernt werden.

2. Verfahren nach Anspruch 1, wobei es sich bei den Mikropartikeln um feste pharmazeutische Wirkstoffe handelt, die eine mittlere Teilchengröße von etwa 0,5 bis etwa 10 µm aufweisen.

3. Verfahren nach einem der Ansprüche 1 oder 2, wobei es sich bei dem Tc*-Salz um NaTc*O₄, ^{99m}Tc-TPAC (Tetraphenylarsomiumpertechnetat) oder ^{99m}Tc-DTPA (Diethylenetriaminepentaacetacid), gegebenenfalls im Gemisch mit NaCl handelt.

4. Verfahren nach Anspruch 1, 2 oder 3, **dadurch gekennzeichnet, dass** das Lösungsmittel ausgewählt ist aus der Gruppe der Alkohole, Ether, Ketone, Halogenkohlenwasserstoffe, der polaren organische Lösungsmittel oder Gemische der vorstehend genannten Lösungsmittel.

5. Verfahren nach Anspruch 1, 2, 3 oder 4, **dadurch gekennzeichnet, dass** das Suspensionsmittel ausgewählt ist aus der Gruppe der unpolaren, aprotonischen Lösungsmittel.

6. Verfahren nach einem der Ansprüche 1 bis 5, **dadurch gekennzeichnet, dass** das Verhältnis von Lösungsmittel zu Suspensionsmittel in einem Bereich zwischen 1:50 und 1:1000, bevorzugt in einem Bereich zwischen etwa 1:100 und 1:500 liegt.

7. Verfahren nach einem der Ansprüche 1 bis 6, **dadurch gekennzeichnet, dass** zur Darstellung der erfindungsgemäßen radioaktiv markierten Mikropartikel pharmazeutische Wirkstoffe zum Einsatz gelangen, die ausgewählt sind aus der Gruppe der Anticholinergika, Betamimetica, Dopaminagonisten, Antiallergika, Leukotrien-Antagonisten und Corticosteroiden, sowie gegebenenfalls Wirkstoffkombinationen davon.

8. Verfahren nach Anspruch 7, **dadurch gekennzeichnet, dass** zur Darstellung der erfindungsgemäßen radioaktiv markierten Mikropartikel Anticholinergika zum Einsatz gelangen, die ausgewählt sind aus der Gruppe der Tiotropiumsalze, Ipratropiumsalze, Oxitropiumsalze,
sowie der Salze der Verbindungen
N-Methyl-2,2-Diphenylpropionsäuretropenolester,
N-Methyl-2,2-Diphenylpropionsäurescopinester,
N-Methyl-2-Fluor-2,2-Diphenylessigsäurescopinester und
N-Methyl-2-Fluor-2,2-Diphenylessigsäuretropenolester
N-Methyl-3,3',4,4'-Tetrafluorbenzilsäuretropenolester,
N-Methyl-3,3',4,4'-Tetrafluorbenzilsäurescopinester,
N-Methyl-4,4'-Dichlorbenzilsäurescopinester,
N-Methyl- 4,4'-Difluorbenzilsäurescopinester,
N-Methyl-3,3'-Difluorbenzilsäuretropenolester,
N-Methyl- 3,3'-Difluorbenzilsäurescopinester und
N-Ethyl-4,4'-Difluorbenzilsäuretropenolester
gegebenenfalls in Form ihrer Hydrate und Solvate.

9. Verfahren nach Anspruch 7, **dadurch gekennzeichnet, dass** zur Darstellung der erfindungsgemäßen radioaktiv markierten Mikropartikel Betamimetika zum Einsatz gelangen, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salbutamol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol und 1-(4-Ethoxycarbonylamino-3-cyano-5-fluorophenyl)-2-(tert.-butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate.

10. Verfahren nach Anspruch 7, **dadurch gekennzeichnet, dass** zur Darstellung der erfindungsgemäßen radioaktiv markierten Mikropartikel Corticosteroide zum Einsatz gelangen, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone.

11. Verfahren nach Anspruch 7, **dadurch gekennzeichnet, dass** zur Darstellung der erfindungsgemäßen radioaktiv markierten Mikropartikel Dopamin-Agonisten zum Einsatz gelangen, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan.

12. Verfahren nach Anspruch 7, **dadurch gekennzeichnet, dass** zur Darstellung der erfindungsgemäßen radioaktiv markierten Mikropartikel Antiallergika zum Einsatz gelangen, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin.

13. Mit dem Technetium-Isotop ^{99m}Tc (Tc*) radioaktiv markierte Mikropartikel als Wirkstoffe ausgewählt aus der Gruppe der Tiotropiumsalze, Ipratropiumsalze, Oxitropiumsalze, sowie der Salze der Verbindungen
N-Methyl-2,2-Diphenylpropionsäuretropenolester,
N-Methyl-2,2-Diphenylpropionsäurescopinester,
N-Methyl-2-Fluor-2,2-Diphenylessigsäurescopinester und
N-Methyl-2-Fluor-2,2-Diphenylessigsäuretropenolester
N-Methyl-3,3',4,4'-Tetrafluorbenzilsäuretropenolester,
N-Methyl-3,3',4,4'-Tetrafluorbenzilsäurescopinester;
N-Methyl-4,4'-Dichlorbenzilsäurescopinester,
N-Methyl-4,4'-Difluorbenzilsäurescopinester,
N-Methyl-3,3'-Difluorbenzilsäuretropenolester,
N-Methyl-3,3'-Difluorbenzilsäurescopinester und
N-Ethyl-4,4'-Difluorbenzilsäuretropenolester gegebenenfalls in Form ihrer Hydrate und Solvate erhältlich nach einem Verfahren gemäß einem der Ansprüche 1 bis 12 im Gemisch mit einem oder mehreren geeigneten, physiologisch unbedenklichen Hilfsstoffen.

14. Verwendung von mit dem Technetium-Isotop ^{99m}Tc (Tc*) radioaktiv markierten Mikropartikeln im Gemisch mit einem oder mehreren geeigneten, physiologisch unbedenklichen Hilfsstoffen nach Anspruch 13 zur Herstellung eines inhalativ applizierbaren Inhalationspulvers.

## Claims

1. Process for preparing microparticles which have a mean particle size of about 0.5 to about 10 µm radioactively labelled with the technetium isotope ^{99m}Tc (Tc*), which is **characterised in that** a Tc* salt is taken up in a solvent, this solution is combined and mixed with microparticles suspended in a suspension agent and finally the suspension agent and the solvent are eliminated by evaporation.

2. Process according to claim 1, wherein the microparticles are solid pharmaceutical active subsances which have a mean particle size of about 0.5 to about 10 µm.

3. Process according to one of claims 1 or 2, wherein the Tc* salt is NaTc*O₄, ^{99m}Tc-TPAC (tetraphenylarsomium pertechnetate) or ^{99m}Tc-DTPA (diethylenetriaminepentaacetic acid), optionally mixed with NaCl.

4. Process according to claim 1, 2 or 3, **characterised in that** the solvent is selected from among the alcohols, ethers, ketones, halohydrocarbons, the polar organic solvents or mixtures of the abovementioned solvents.

5. Process according to claim 1, 2, 3 or 4, **characterised in that** the suspension agent is selected from among the nonpolar aprotic solvents.

6. Process according to one of claims 1 to 5, **characterised in that** the ratio of solvent to suspension agent is in a range between 1:50 and 1:1000, preferably in a range between about 1:100 and 1:500.

7. Process according to one of claims 1 to 6, **characterised in that** in order to prepare the radioactively labelled microparticles according to the invention pharmaceutical active substances are used which are selected from among the anticholinergics, betamimetics, dopamine agonists, antiallergics, leukotriene antagonists and corticosteroids, and optionally active substance combinations thereof.

8. Process according to claim 7, **characterised in that** in order to prepare the radioactively labelled microparticles according to the invention anticholinergics are used which are selected from among the tiotropium salts, ipratropium salts, oxitropium salts, as well as the salts of the compounds
tropenol N-methyl-2,2-diphenylpropionate,
scopine N-methyl-2,2-diphenylpropionate,
scopine N-methyl-2-fluoro-2,2-diphenylacetate and
tropenol N-methyl-2-fluoro-2,2-diphenylacetate
tropenol N-methyl-3,3',4,4'-tetrafluorobenzilate,
scopine N-methyl-3,3',4,4'-tetrafluorobenzilate;
scopine N-methyl-4,4'-dichlorobenzilate,
scopine N-methyl- 4,4'-difluorobenzilate,
tropenol N-methyl-3,3'-difluorobenzilate,
scopine N-methyl-3,3'-difluorobenzilate and
tropenol N-ethyl-4,4'-difluorobenzilate,
optionally in the form of the hydrates and solvates thereof.

9. Process according to claim 7, **characterised in that** in order to prepare the radioactively labelled microparticles according to the invention betamimetics are used which are selected from among the group consisting of bambuterol, bitolterol, carbuterol, clenbuterol, fenoterol, formoterol, hexoprenaline, ibuterol, pirbuterol, procaterol, reproterol, salbutamol, salmeterol, sulphonterol, terbutaline, tolubuterol, 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluoromethylphenyl)-2-*tert*.-butylamino)ethanol and 1-(4-ethoxycarbonylamino-3-cyano-5-fluorophenyl)-2-(*tert*.-butylamino)ethanol, optionally in the form of their racemates, their enantiomers, their diastereomers, as well as optionally the pharmacologically acceptable acid addition salts and hydrates thereof.

10. Process according to claim 7, **characterised in that** in order to prepare the radioactively labelled microparticles according to the invention corticosteroids are used which are selected from among flunisolide, beclomethasone, triamcinolone, budesonide, fluticasone, mometasone, ciclesonide, rofleponide, GW 215864, KSR 592, ST-126 and dexamethasone.

11. Process according to claim 7, **characterised in that** in order to prepare the radioactively labelled microparticles according to the invention dopamine agonists are used which are selected from among bromocriptine, cabergolin, alpha-dihydroergocryptine, lisuride, pergolide, pramipexol, roxindol, ropinirol, talipexol, tergurid and viozan.

12. Process according to claim 7, **characterised in that** in order to prepare the radioactively labelled microparticles according to the invention antiallergics are used which are selected from among epinastin, cetirizin, azelastin, fexofenadin, levocabastin, loratadine, mizolastin, ketotifen, emedastin, dimetinden, clemastine, bamipin, cexchloropheniramine, pheniramine, doxylamine, chlorophenoxamine, dimenhydrinate, diphenhydramine, promethazine, ebastin,
desloratidine and meclizine.

13. Microparticles radioactively labelled with the technetium isotope ^{99m}Tc (Tc*) as active substances, selected from among the tiotropium salts, ipratropium salts, oxitropium salts, as well as the salts of the compounds
tropenol N-methyl-2,2-diphenylpropionate,
scopine N-methyl-2,2-diphenylpropionate,
scopine N-methyl-2-fluoro-2,2-diphenylacetate and
tropenol N-methyl-2-fluoro-2,2-diphenylacetate
tropenol N-methyl-3,3',4,4'-tetrafluorobenzilate,
scopine N-methyl-3,3',4,4'-tetrafluorobenzilate;
scopine N-methyl-4,4'-dichlorobenzilate,
scopine N-methyl-4,4'-difluorobenzilate,
tropenol N-methyl-3,3'-difluorobenzilate,
scopine N-methyl- 3,3'-difluorobenzilate and
tropenol N-ethyl-4,4'-difluorobenzilate,
optionally in the form of the hydrates and solvates thereof, which may be obtained by a process according to one of claims 1 to 12, in admixture with one or more suitable physiologically acceptable excipients.

14. Use of microparticles radioactively labelled with the technetium isotope ^{99m}Tc (Tc*) in admixture with one or more suitable physiologically acceptable excipients according to claim 13, for preparing an inhalable powder which may be administered by inhalation.

## Revendications

1. Procédé de production de microparticules à marquage radioactif par l'isotope de technétium ^{99m}Tc (Tc*) présentant une taille de particules moyenne d'environ 0,5 à environ 10 µm, **caractérisé en ce qu'**un sel de Tc* est absorbé dans un solvant, cette solution étant ajoutée et mélangée à des microparticules en suspension dans un milieu de suspension et le milieu de suspension et le solvant étant ensuite éliminés par évaporation.

2. Procédé selon la revendication 1, dans lequel les microparticules sont des substances pharmaceutiques solides qui présentent une taille de particules moyenne d'environ 0,5 à environ 10 µm.

3. Procédé selon l'une des revendications 1 ou 2, dans lequel le sel de Tc* est le NaTc*O₄, le ^{99m}Tc-TPAC (tétraphénylarsomiumpertechnétate) ou le ^{99m}Tc-DTPA (diéthylènetriaminepenta-acétacide), éventuellement en mélange avec du NaCl.

4. Procédé selon la revendication 1, 2 ou 3, **caractérisé en ce que** le solvant est choisi dans le groupe comprenant les alcools, les éthers, les cétones, les hydrocarbures halogénés, les solvants organiques polaires ou les mélanges des solvants précédemment cités.

5. Procédé selon la revendication 1, 2, 3 ou 4, **caractérisé en ce que** le milieu de suspension est choisi dans le groupe comprenant des solvants non polaires aprotoniques.

6. Procédé selon l'une des revendications 1 à 5, **caractérisé en ce que** le rapport du solvant au milieu de suspension se situe dans une plage entre 1 : 50 et 1 : 1000, de préférence, dans une plage entre environ 1 : 100 et 1 : 500.

7. Procédé selon l'une des revendications 1 à 6, **caractérisé en ce que** l'on utilise comme illustration des microparticules à marquage radioactif selon l'invention, des substances pharmaceutiques qui sont choisies dans le groupe comprenant les anti-cholinergiques, les bêtamimétiques, les agonistes dopaminergiques, les anti-allergiques, les antagonistes des leucotriènes et les corticostéroïdes, et éventuellement, des combinaisons de principes actifs de ceux-ci.

8. Procédé selon la revendication 7, **caractérisé en ce que** comme illustration des microparticules à marquage radioactif selon l'invention, on utilise des anti-cholinergiques qui sont choisis dans le groupe des sels de tiotropium, des sels d'ipratropium, des sels d'oxytropium et des sels des composés
✔ ester de tropénol d'acide N-méthyl-2,2-diphénylpropionique
✔ ester de scopine d'acide N-méthyl-2,2-diphénylpropionique
✔ ester de scopine d'acide N-méthyl-2-fluoro-2,2-diphénylacétique et
✔ ester de tropénol d'acide N-méthyl-2-fluoro-2,2-diphénylacétique
✔ ester de tropénol d'acide N-méthyl-3,3',4,4'-tétrafluorobenzylique
✔ ester de scopine d'acide N-méthyl-3,3',4,4'-tétrafluorobenzylique
✔ ester de scopine d'acide N-méthyl-4,4'-dichlorobenzylique,
✔ ester de scopine d'acide N-méthyl-4,4'-difluorobenzylique,
✔ ester de tropénol d'acide N-méthyl-3,3'-difluorobenzylique,
✔ ester de scopine d'acide N-méthyl-3,3'-difluorobenzylique et
✔ ester de tropénol d'acide N-éthyl-4,4'-difluorobenzylique
✔ éventuellement sous la forme de leurs hydrates et solvates.

9. Procédé selon la revendication 7, **caractérisé en ce que**, comme illustration des microparticules marquées radioactivement selon l'invention, on utilise des bêtamimétiques qui sont choisis dans le groupe comprenant le bambutérol, le bitoltérol, le carbutérol, le clenbutérol, le fénotérol, le formotérol, l'hexoprénaline, l'ibutérol, le pirbutérol, le procatérol, le réprotérol, le salbutamol, le salmétérol, le sulfontérol, la terbutaline, le tolubutérol, la 4-hydroxy-7-[2-{[2-{[3-(2-phényléthoxy)propyl]sulfonyl}éthyl]-amino}éthyl]-2(3H)-benzothiazolone, le 1-(2-fluoro-4-hydroxyphényl)-2-[4-(1-benzimidazolyl)-2-méthyl-2-butylamino]éthanol, le 1-[3-(4-méthoxybenzyl-amino)-4-hydroxyphényl]-2-[4-(1-benzimidazolyl)-2-méthyl-2-butylamino]éthanol, le 1-[2H-5-hydroxy-3-oxo-4H-1,4-benzoxazin-8-yl]-2-[3-(4-N,N-diméthylamino-phényl)-2-méthyl-2-propylaminol]éthanol, le 1-[2H-5-hydroxy-3-oxo-4H-1,4-benzoxazin-8-yl]-2-[3-(4-méthoxyphényl)-2-méthyl-2-propylamino]éthanol, le 1-[2H-5-hydroxy-3-oxo-4H-1,4-benzoxazin-8-yl]-2-[3-(4-n-butyloxyphényl)-2-méthyl-2-propylaminoléthanol, le 1-[2H-5-hydroxy-3-oxo-4H-1,4-benzoxazin-8-yl]-2-{4-[3-(4-methoxyphényl)-1,2,4-triazol-3-yl]-2-méthyl-2-butylamino}éthanol, le 5-hydroxy-8-(1-hydroxy-2-isopropylaminobutyl)-2H-1,4-benzoxazin-3-(4H)-one, le 1-(4-amino-3-chloro-5-trifluorométhylphényl)-2-tert-butylamino)éthanol et le 1-(4-éthoxycarbonylamino-3-cyano-5-fluorophényl)-2-(tert-butylamino)éthanol, éventuellement sous forme de leurs racémates, de leurs énantiomères, de leurs diastéréomères et éventuellement de leurs sels d'addition acides et hydrates pharmacologiquement acceptables.

10. Procédé selon la revendication 7, **caractérisé en ce que**, comme illustration des microparticules marquées radioactivement selon l'invention, on utilise des corticostéroïdes qui sont choisis dans le groupe comprenant le flunisolide, la béclométhasone, le triamcinolone, le budésonide, la fluticasone, la mométasone, le ciclésonide, le rofléponide, le GW 215864, le KSR 592, le ST-126 et la dexamétasone.

11. Procédé selon la revendication 7, **caractérisé en ce que**, comme illustration des microparticules marquées radioactivement selon l'invention, on utilise des agonistes dopaminergiques qui sont choisis dans le groupe comprenant la bromocriptine, la cabergline, l'alphadihydro-ergocryptine, le lisuride, le pergolide, le pramipexol, le roxindol, le ropinirol, le talipexol, le terguride et le viozan.

12. Procédé selon la revendication 7, **caractérisé en ce que**, comme illustration des microparticules marquées radioactivement selon l'invention, on utilise des anti-allergiques qui sont choisis dans le groupe comprenant l'épinastine, la cétirizine, l'azélastine, la fexofénadine, la lévocabastine, la loratadine, la mizolastine, le cétotifène, l'émédastine, le dimétindène, la clémastine, la bamipine, le cexchlorophéniramine, la phéniramine, la doxylamine, la chlorophénoxamine, le dimenhydrinate, la diphenhydramine, la prométhazine, l'ébastine, la desloratidine et la méclozine.

13. Microparticule à marquage radioactif par l'isotope de technétium ^{99m}Tc (Tc*) comme principes actifs, choisie dans le groupe comprenant les sels de tiotropium, des sels d'ipratropium, des sels d'oxytropium et des sels des composés ✔ ester de tropénol d'acide N-méthyl-2,2-diphénylpropionique
✔ ester de scopine d'acide N-méthyl-2,2-diphénylpropionique
**✔** ester de scopine d'acide N-méthyl-2-fluoro-2,2diphénylacétique et
✔ ester de tropénol d'acide N-méthyl-2-fluoro-2,2-diphénylacétique
✔ ester de tropénol d'acide N-méthyl-3,3',4,4'-tétrafluorobenzylique
✔ ester de scopine d'acide N-méthyl-3,3',4,4'-tétrafluorobenzylique
✔ ester de scopine d'acide N-méthyl-4,4'-dichlorobenzylique,
✔ ester de scopine d'acide N-méthyl-4,4'-difluorobenzylique,
✔ ester de tropénol d'acide N-méthyl-3,3'-difluorobenzylique,
✔ ester de scopine d'acide N-méthyl-3,3'-difluorobenzylique et
✔ ester de tropénol d'acide N-éthyl-4,4'-difluorobenzylique
✔ éventuellement sous la forme de leurs hydrates et solvates, pouvant être obtenus selon un procédé selon l'une des revendications 1 à 12 en mélange avec un ou plusieurs adjuvants appropriés, physiologiquement acceptables.

14. utilisation de microparticules à marquage radioactif par l'isotope de technétium ^{99m}Tc (Tc*) en mélange avec un ou plusieurs adjuvants appropriés, physiologiquement acceptables selon la revendication 13, pour la production d'une poudre pour inhalation pouvant être appliquée par inhalation.
